(19) Europäisches Patentamt
European Patent Office
Office européen des brevets

(11) Publication number: **0 171 216**
**B1**

(12) **EUROPEAN PATENT SPECIFICATION**

(45) Date of publication of patent specification: **30.11.88**

(51) Int. Cl.$^4$: **C 07 C 45/67,** C 07 C 47/21

(21) Application number: **85305146.4**

(22) Date of filing: **18.07.85**

(00) **A request pursuant to Rule 88 EPC for correction of the description was filed on 24.07.1985.**

(54) **A method for the production of tiglic aldehyde.**

(30) Priority: **24.07.84 JP 154635/84**

(43) Date of publication of application:
**12.02.86 Bulletin 86/07**

(45) Publication of the grant of the patent:
**30.11.88 Bulletin 88/48**

(84) Designated Contracting States:
**CH DE FR GB LI**

(56) References cited:
**FR-A-2 028 390**
**FR-A-2 386 509**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Matsuda, Teruo**
**No. 8-6, Hoshigoe-cho**
**Niihama-shi Ehime (JP)**
Inventor: **Shimizu, Shinkichi**
**18-14, Fujisakamoto-machi 3-chome**
**Hirakata-shi Osaka (JP)**
Inventor: **Iwasa, Masaaki**
**No. 4-13, Hoshigoe-cho**
**Niihama-shi Ehime (JP)**

(74) Representative: **Diamond, Bryan Clive et al**
**Gee & Co. Chancery House Chancery Lane**
**London WC2A 1QU (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 171 216 B1

**Description**

This invention relates to a novel method for the production of tiglic aldehyde

[2-methyl-2-butene-1-al, 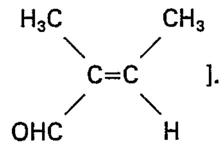 ].

Tiglic aldehyde is an important raw material for the synthesis of isoterpenes. Tiglic acid

[ 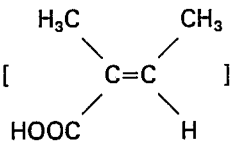 ]

which is obtained by oxidation of tiglic aldehyde is useful as a raw material for the synthesis of driers for perfumes, varnishes, inks, and the like. Further, a chloride of tiglic aldehyde

[4-chloro-2-methyl-2-butene-1-al, 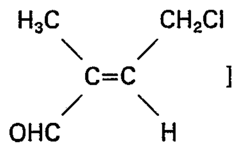 ]

is a useful compound to extend an isoterpene chain by one unit.

Hitherto, as a method for the production of tiglic aldehyde, a so-called Cross's aldol condensation method wherein acetaldehyde and propionaldehyde are condensed in a strong basic condition is known. However, this method has a drawback that the yield of said aldehyde is poor with a large quantity of various by-products being formed (see M. B. Green, W. T. Hickinbottom, *Journal of Chemical Society,* page 3262 (1975)).

An object of this invention is to provide a process for producing tiglic aldehyde, especially such process which provides a good yield from materials which are available at a low price.

FR—A—2028390 (=GB—A—1286031) discloses a process of isomerizing a buten-2-ol in the liquid phase in the presence of hydrogen and of palladium or a compound of palladium as catalyst. Example 5 discloses the isomerization of 4-acetoxy-2-methylene-butanol-1, but the yield is only 30% based on the starting material.

FR—A—2386509 (=GB—A—1597876-7) discloses a process of isomerizing 3-methyl-3-butenol in the presence of a catalyst such as an alkaline earth metal compound; the starting material can be prepared by dehydrogenation of 3-methyl-3-buten-1-ol in the presence as catalyst of a strong acid or a basic compound, e.g. compounds of alkali metals or zinc. This is not a hydrogenation process and palladium or similar group VIII catalysts are not used.

We have now found a type of catalyst which is more efficient for the desired specific hydrogenation isomerisation of 2-methylene-butanol.

According to the present invention we provide a process for producing tiglic aldehyde, which comprises isomerizing ethylacrolein in a liquid phase in the presence of hydrogen gas and a hydrogenation catalyst poisoned by a sulfur-containing compound which is selected from sodium dithionite, sulfonic acid, thiophene, thiourea and 1,1,3,3-tetramethylthiourea.

The reaction equation is

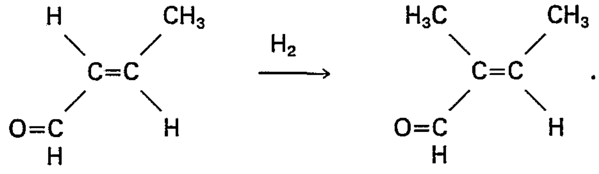


The ethylacrolein used as starting material for the synthesis of tiglic aldehyde in this invention can be almost quantitatively prepared by condensing the readily available industrial materials, n-butylaldehyde and a formalin aqueous solution (see U.S. Patent 4,346,239).

In the liquid phase reaction of the invention for the production of tiglic aldehyde, ethaylacrolein is

heated in the copresence of a solvent and a catalyst. Examples of the solvent which can be used in this reaction include aromatic hydrocarbons, alcohols and aliphatic hydrocarbons. Illustrative of such solvents are toluene, ethylbenzene, xylene, n-butanol and n-octane.

Examples of the catalyst which can be used in this reaction include metals belonging to the Group VIII of the periodic table, such as platinum, palladium, rhodium or ruthenium. These metals are generally used supported on a carrier such as an activated carbon or alumina. These supported catalysts are known for use as a hydrogenation catalyst and are commercially available, for example, 5% palladium-on-carbon and 0.5% palladium-on-alumina made by Nippon Engelhard Co., Ltd.

The catalyst is poisoned by sodium dithionite, sulfuric acid, thiophene, thiourea or 1,1,3,3-tetramethylthiourea; its reactivity is thereby reduced but the selectivity of the isomerization increases.

Since hydrogen is copresent in the reaction system, a saturated aldehyde, 2-methylbutanal

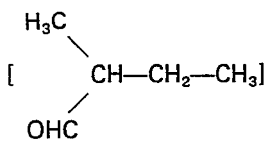

is formed as a by-product, but the isomerization becomes extremely active, leading to an increase in yield over prior known processes.

The reaction temperature may be 50°C or higher, but when the reaction temperature is too low, the rate of reaction is low, whereas when the reaction temperature is too high, a loss which may be caused by the polymerization occurs. Accordingly, preferable results can be obtained in the range of from 80°C to 150°C.

The catalyst used can be repeatedly used ten times after mere recovering by filtration without any other treatment, and there was not found any change in its reactivity.

Tiglic aldehyde can thus be prepared in a good yield from n-butylaldehyde and formalin aqueous solution as materials for making the ethylacrolein used.

The following examples are illustrative of this invention. The catalysts and other specific ingredients and processing parameters are presented as being typical, but modifications can be made therefrom within the scope of the invention.

Preparation of catalysts

Catalyst 1

0.052 g of sodium dithionite ($Na_2S_2O_4$) was dissolved in 100 ml of water, and 20.0 g of a 5% palladium-on-carbon powder (Nippon Engel Hard Co., Ltd.) was added thereto. The mixture was then stirred for 30 minutes. The mixture was heated on a sand bath to evaporate off water, and the residue was kept in a vacuum drying oven at 120°C for 24 hours.

Catalyst 2

0.5 g of thiourea was dissolved in 300 ml of acetone, and 20 g of a 5% palladium-on-carbon powder (Nippon Engel Hard Co., Ltd.) was added thereto. The mixture was then stirred for 30 minutes. The acetone was evaporated off by a rotary evaporator, and the residue was kept in a vacuum drying oven at 120°C for 12 hours.

Catalyst 3

0.4 g of thiophene was dissolved in 300 ml of acetone, and 20 g of a 5% palladium-on-carbon powder was added thereto. The mixture was then stirred at room temperature for 30 minutes. The acetone was evaporated off by a rotary evaporator, and the residue was kept in a vacuum drying oven at 120°C for 12 hours.

Example 1

In a stainless steel reactor having a capacity of 100 ml were placed 8.4 g of ethylacrolein (0.1 mol), 25 ml of ethylbenzene, and 0.3 g of catalyst 1. The reactor was purged with nitrogen, and 500 ml of hydrogen gas at standard condition was then introduced in the reactor. The mixture was heated at 100°C for 6 hours.

After cooling the reactor, the reaction solution was analyzed by gas chromatography. The results of the reaction showed that the conversion of ethylacrolein was 97%, the yield of tiglic aldehyde was 74% and the yield of saturated aldehyde (2-methylbutane-1-al) was 22%.

The identification of the product obtained relied on mass analysis and NMR analysis.

Examples 2 to 6

Using catalyst 1 or 2, the same procedure as in Example 1 were followed except for changing the kind of the solvent (but not changing the amount (weight) of the solvent). The results are shown in Table 1.

TABLE 1

| Example No. | Solvent | Catalyst | Hydrogen (NTP) (ml) | Reaction condition temperature (°C) ×time (hr) | Conversion of Ethylacrolein (%) | Yield of tiglic aldehyde (%) | Yield of saturated aldehyde (%) |
|---|---|---|---|---|---|---|---|
| 2 | toluene | catalyst 2 | 680 | 100×6 | 95 | 63 | 29 |
| 3 | methanol | catalyst 2 | 680 | 100×6 | 80 | 48 | 16 |
| 4 | n-butanol | catalyst 2 | 280 | 120×4 | 82 | 59 | 15 |
| 5 | n-octane | catalyst 2 | 280 | 120×4 | 79 | 63 | 14 |
| 6 | xylene | catalyst 1 | 400 | 100×6 | 85 | 67 | 18 |

Example 7

The catalyst used in Example 1 was filtered and then reused. The reaction was carried out at 100°C for 6 hours using 4.2 g of ethylacrolein, 12.5 ml of ethylbenzene, 0.15 g of recovered catalyst 1, and 250 ml of hydrogen gas. The results are shown in Table 2.

TABLE 2

| Solvent | Catalyst | Hydrogen (NTP) (ml) | Reaction condition temperature (°C) ×time (hr) | Conversion of ethylacrolein (%) | Yield of tiglic aldehyde (%) | Yield of saturated aldehyde (%) |
|---|---|---|---|---|---|---|
| ethylbenzene | catalyst 1 | 250 | 100×6 | 99 | 75 | 22 |

Example 8

In a glass reactor equipped with a reflux condenser were placed 25 ml of toluene and 8.4 g of ethylacrolein, the reactor was purged with nitrogen, and 0.3 g of catalyst 3 was then added thereto. The mixture was refluxed under heating for about 6 hours while supplying a 5% hydrogen gas (the balance being nitrogen) into the reactor at a flow rate of 30 ml/min. The results are shown in Table 3.

TABLE 3

| Reaction time (hr) | Conversion of ethylacrolein (%) | Yield of tiglic aldehyde (%) | Yield of 2-methylbutane-1-al (%) |
|---|---|---|---|
| 6 | 49 | 39 | 7 |

Example 9

In a glass reactor was packed 2 g of a 5% palladium-on-alumina catalyst and ethylacrolein, hydrogen and nitrogen were introduced thereinto at a ratio of 1/0.037/3.4 in the gas phase. The yield of tiglic aldehyde was 35% at a space velocity (SV) of 700 $hr^{-1}$ and at 100°C.

Example 10

The procedure of Example 9 was repeated but without the introduction of the hydrogen and at a temperature of 150°C. The tiglic aldehyde a yield was 36%.

**Claims**

1. A process for producing tiglic aldehyde of the formula:

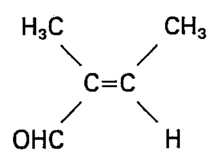

which comprises isomerizing ethylacrolein in a liquid phase in the presence of hydrogen gas and a hydrogenation catalyst poisoned by a sulfur-containing compound which is selected from sodium dithionite, sulfonic acid, thiophene, thiourea and 1,1,3,3-tetramethylthiourea.

2. A process in accordance with Claim 1, wherein the catalyst is palladium, platinum, rhodium or ruthenium.

**Patentansprüche**

1. Verfahren zur Herstellung von Tiglinaldehyd der Formel,

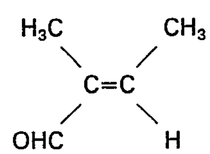

umfassend die Isomerisierung von Aethylacrolein in der flüssigen Phase und in Gegenwart von Wasserstoff Gas und von einem Hydrierungskatalysator, der unter Natriumdithionit, Sulfonsäure, Thiophen, Thioharnstoff und 1,1,3,3-Tetramethylthioharnstoff gewählt ist.

2. Verfahren gemäss Anspruch 1, worin der Katalysator Palladium, Platin, Rhodium oder Ruthenium ist.

**Revendications**

1. Procédé pour la production d'aldéhyde tiglique de formule

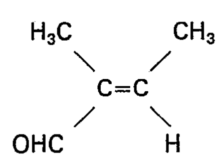

qui comprend l'isomérisation d'éthylacroléine en phase liquide et en présence de gaz hydrogène et d'un catalyseur d'hydrogénation empoisonné par un composé contenant du soufre choisi parmi le dithionite de sodium, l'acide sulfonique, le thiophène, la thiourée et la 1,1,3,3-tétraméthylthiourée.

2. Procédé selon la revendication 1, dans lequel le catalyseur est le palladium, le platine, le rhodium ou le ruthénium.